# EUROPEAN PATENT APPLICATION

(11) **EP 4 789 683 A1**
(43) Date of publication of application: **12.08.2026**
(21) Application number: 24874133.2
(22) Date of filing: 04.10.2024
(51) Int. Cl.: A61K 47/00, A61K 31/7105, C12N 15/113, B82Y 5/00

(54) **NANOSYSTEM FOR USE IN CAPTURING, DIRECTING AND/OR TRANSPORTING BIOLOGICALLY ACTIVE MOLECULES TO ADIPOSE TISSUE**

(30) Priority: 04.10.2023 ES 202330831; 19.01.2024 ES 202430047
(71) Applicant: Servicio Andaluz de Salud, 41071 Sevilla (ES); Universidad De Malaga, E-29071 Málaga (ES); Universidad de Sevilla, 41013 Sevilla (ES); Consorcio Centro de Investigación Biomédica en Red, 28029 Madrid (ES)
(72) Inventor: EL BEKAY RIZKY, Rajaa, 41071 Sevilla (ES); LHAMYANI, Said, 41071 Sevilla (ES); GENTILE, Adriana Mariel, 41071 Sevilla (ES); MENGUAL MESA, María, 41071 Sevilla (ES); BOUAOUDA ARAFA, Adnan, 41071 Sevilla (ES); GIRÁLDEZ PÉREZ, Rosa María, 29071 Málaga (ES); GRUESO MOLINA, Elia María, 41013 Sevilla (ES)
(74) Representative: Hoffmann Eitle
(86) International application number: PCT/ES2024/070605
(87) International publication number: WO 2025/074024

(57) **Abstract**

The present invention relates to a nanosystem for use in capturing, directing and/or transporting biologically active molecules to adipose tissue, therefore serving to treat illnesses such as obesity and/or type 2 diabetes.

## Description

### FIELD OF THE INVENTION

The present invention belongs to the medical field. Particularly, the present invention relates to a nanosystem for use in capturing, directing and/or transporting biologically active molecules to adipose tissue, therefore serving to treat illnesses such as, for example, obesity and/or type 2 diabetes.

### STATE OF THE ART

Adipocytes are found throughout the body, mainly in subcutaneous, visceral and medullary deposits as white adipose tissues (WAT), brown adipose tissues (BAT) or more specialized adipose tissues.

At the cellular level, mature adipocytes comprise only 11 to 40% of the total cell population in adipose tissue, which also contains preadipocytes, stem cells, macrophages, endothelial cells and vasculature, known collectively as vascular stromal cells.

Due to this cellular diversity, one of the main challenges in drug administration is to reach adipose tissue specifically.

Therefore, there is an unmet medical need in designing strategies that allow capturing, directing and/or transporting biologically active molecules specifically to adipose tissue, which would have a great potential to offer a wide variety of therapeutic and imaging diagnostic applications in related illnesses such as the case of obesity and associated metabolic illnesses.

The present invention aims to solve this technical problem and relates to a nanosystem for use in capturing, directing and/or transporting biologically active molecules to adipose tissue.

### DESCRIPTION OF THE INVENTION

### Brief Description of the Invention

The present invention relates to a nanosystem for use in capturing, directing and/or transporting biologically active molecules to adipose tissue.

Particularly, the inventors of the present invention show, as a proof of concept, **Example 1** and **Example 2** relating to the synthesis of a lipophilic and cationic nanosystem with a high positive charge formed by Gemini cationic surfactant-coated gold nanoparticles and a microRNA as an example of a biologically active molecule (Au@16-Ph-16/miR-X mimic). The high overall positive charge on the surface of said nanosystem means that it can be specifically directed to subcutaneous adipose tissue by means of creating a negatively-charged electric field. However, the affinity of the 16-Ph-16 surfactant coating the nanoparticle for adipose tissue stems not only from its high charge, but also from its surface activity. In that sense, earlier studies have demonstrated how the micelle-forming capacity of this surfactant is highly enhanced. This property, along with its low micropolarity and high solubilizing power for hydrophobic compounds, contributes to increasing its affinity for adipose tissue. The negative field was created by means of two methods: i) creating a low-intensity electric field using the electroacupuncture method and using a voltage above the Zeta potential of the nanosystem which, at the same time, is not harmful to the subject to be treated, and ii) by means of laser thermal treatment. These two methods have allowed specifically trapping and localizing the nanosystem in a specific site of the body.

It is important to point out that this optimization in the delivery and directing of the nanosystem allows designing a safe and effective drug therapy specifically targeting localized sites of adipose tissue, preventing the nanosystem from reaching other organs or tissues where fat may be present. This strategy allows both directing the nanosystem to a specific site of the body with adipose tissue and trapping the nanoparticles in the site of application such that they cannot leave the site of application, which in this case would be subcutaneous fat.

In that sense, it is important to point out that the nanosystem of the invention is designed such that it causes adipose tissue to capture it specifically. Obtaining a nanosystem that is captured specifically by adipose tissue confers a significant inventive leap to the invention because, unlike certain tissues or organs having different proteins on the cell surface that may be useful for drug directing, adipose tissue lacks specific markers due to its diverse cellular composition, which includes adipocytes, a complex network of blood vessels, extracellular matrix and other cell types [Y. Onogi, A. E. M. M. Khalil, and S. Ussar, "Identification and characterization of adipose surface epitopes," Biochem J, vol. 477, no. 13, pp. 2509-2541, Jul. 2020, doi: 10.1042/BCJ20190462*].* This complexity means that it is a challenge to design directing strategies for the exclusive and efficient delivery of biologically active substances to adipose tissue, while preventing off-target effects in other tissues.

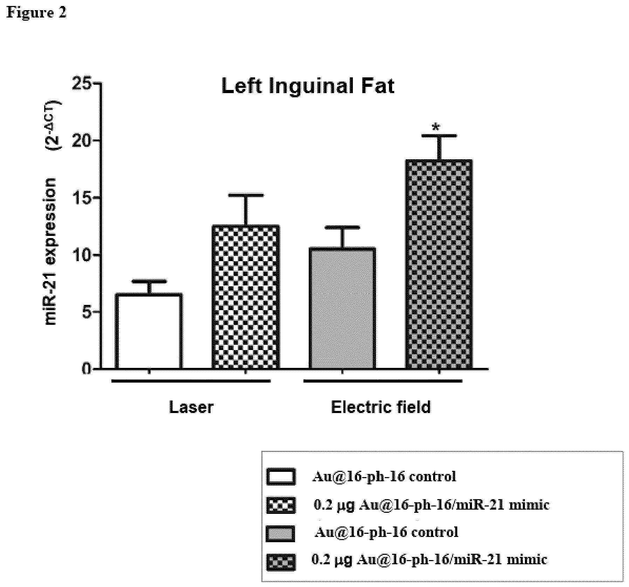

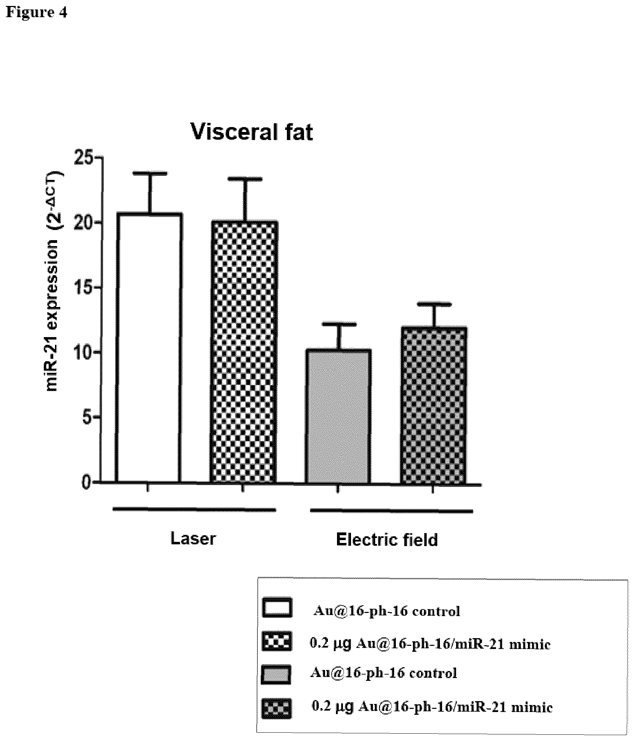

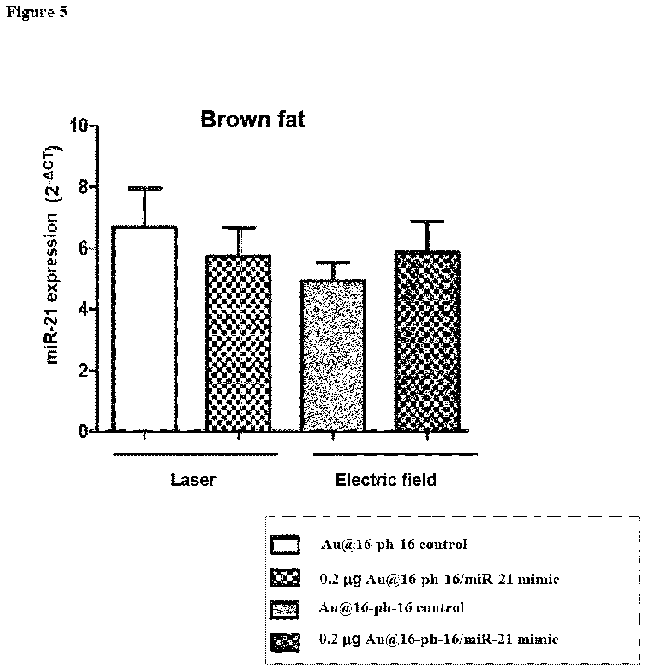

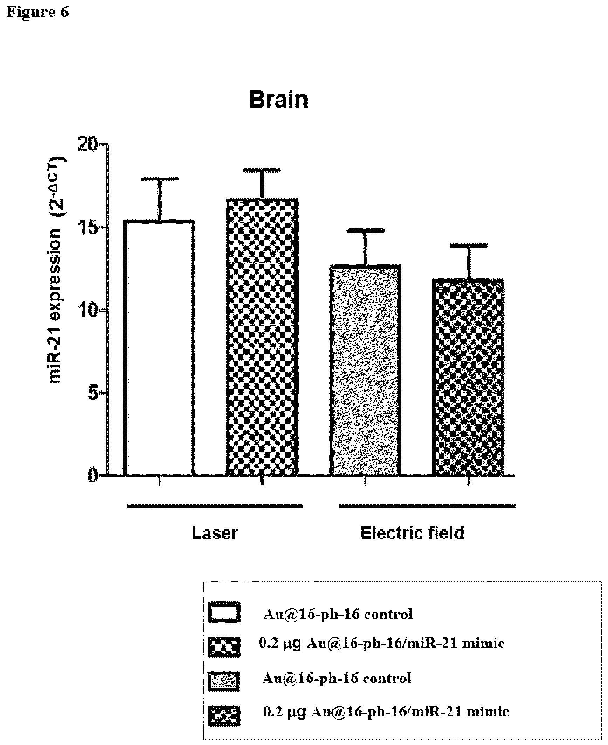

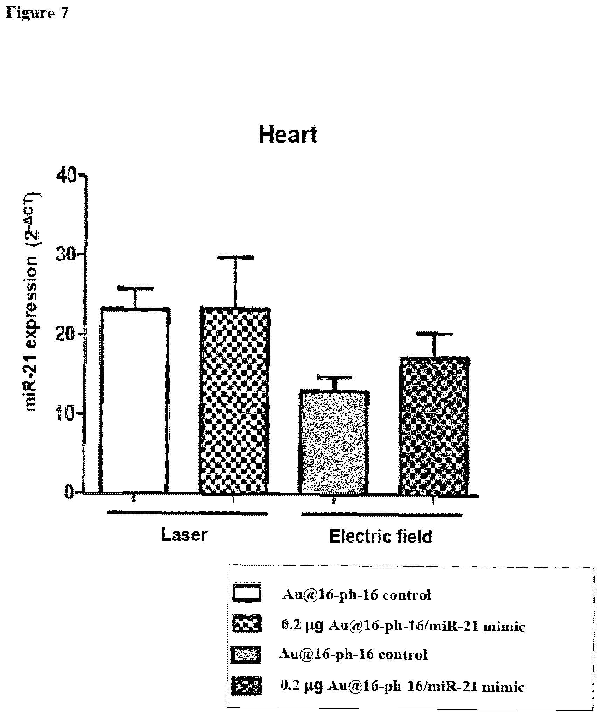

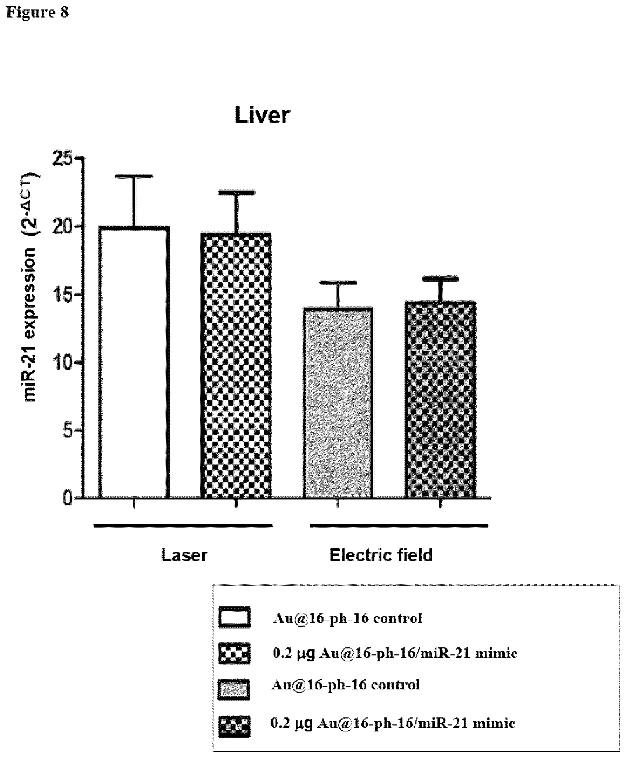

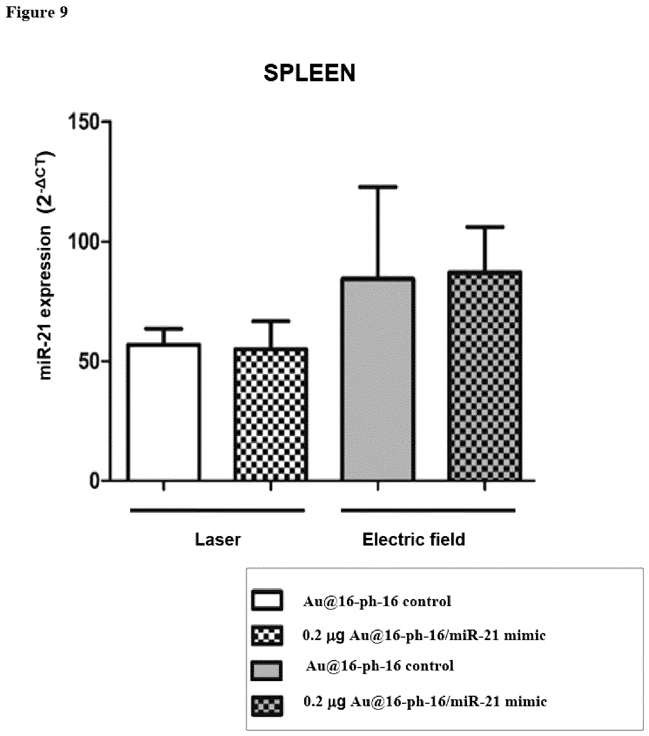

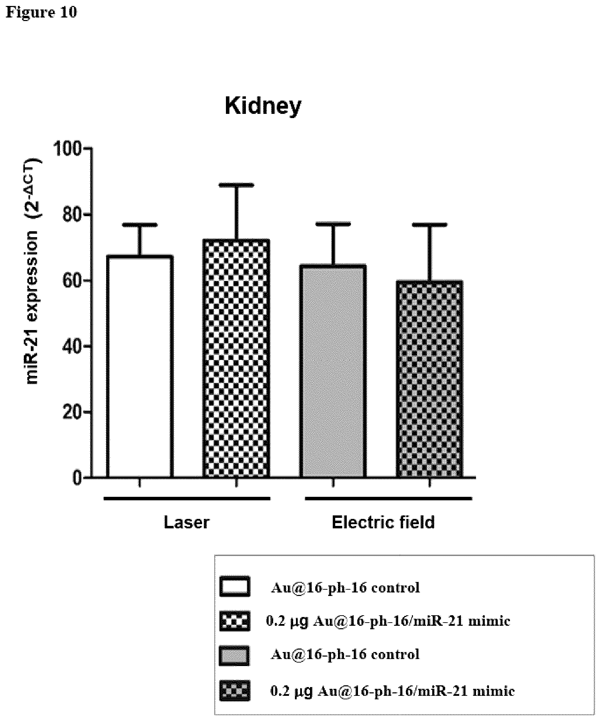

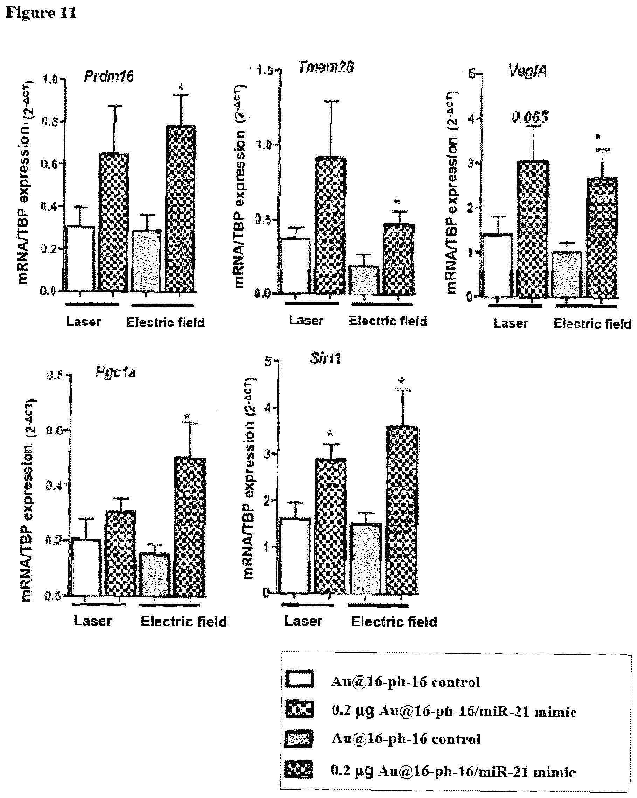

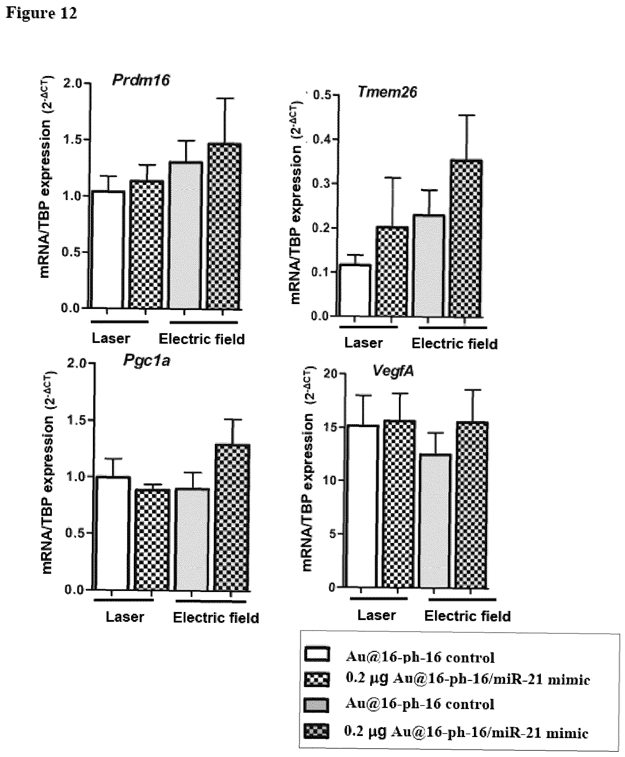

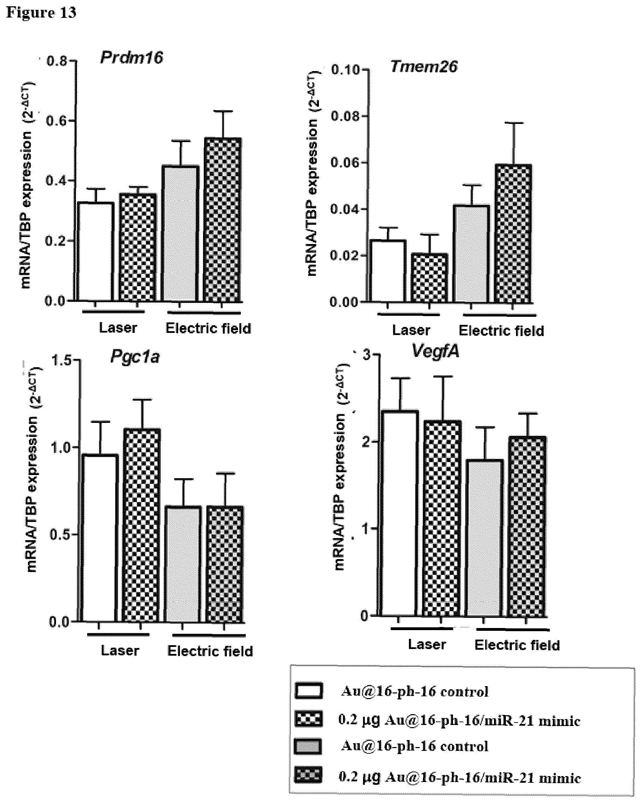

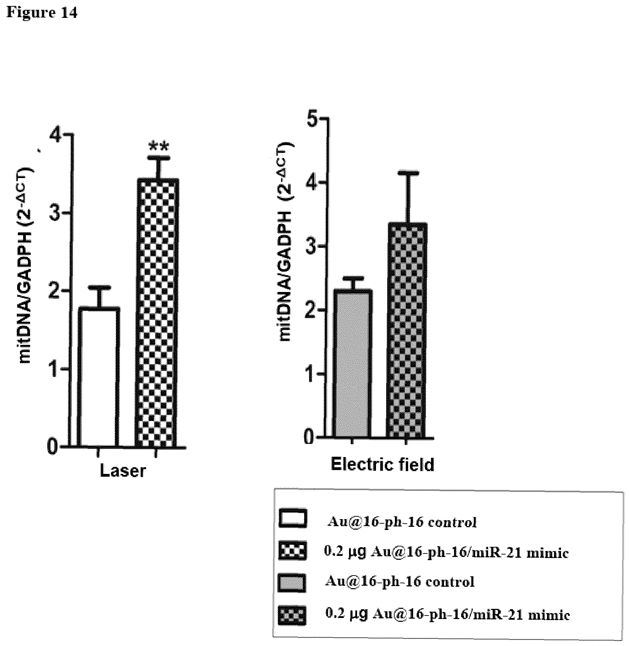

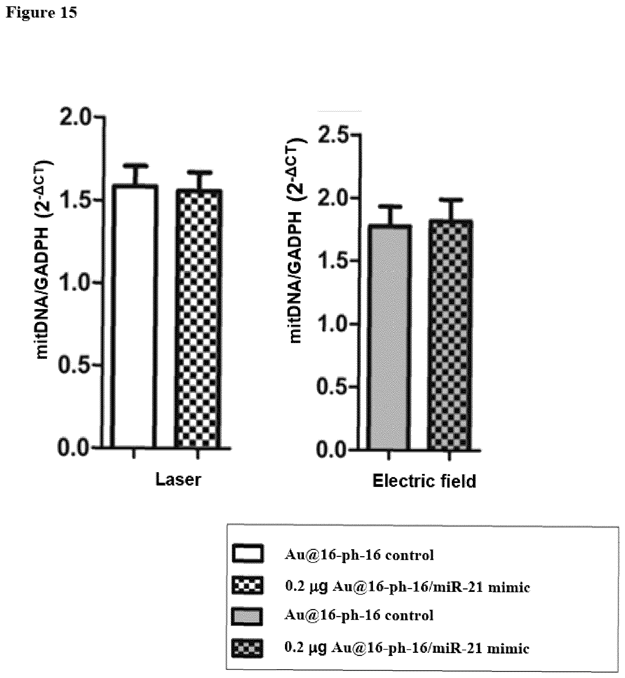

The results obtained (see **Example 2** and **Figures 2** to **10**) demonstrate a significant increase of the biologically active molecule injected only in the site where the nanosystem was injected and where both the electric field and laser were applied. Meanwhile, no significant modification in the levels of the assayed biologically active substance (miR-21) was observed in the other tissues. Particularly, **Figures 11** to **15** show the analysis of the effect of the nanosystem on browning-mediating parameters, showing that marker induction was detected significantly in the left inguinal fat where puncture was performed and where the two drive systems, i.e., laser and electric field, were applied.

Therefore, the first aspect of the present invention relates to a nanosystem for use in capturing, directing and/or transporting biologically active molecules to adipose tissue, comprising: a) a positively-charged nanoparticle attached to a biologically active molecule, and b) an irradiation system that confers a negative charge specifically on adipose tissue, causing the nanoparticle to be specifically directed to and captured by the adipose tissue as a result of a charge difference.

In a preferred aspect, the positively-charged nanoparticle is attached to the biologically active molecule through cationic surfactants that improve the uptake and stability of the biologically active molecule.

In a preferred aspect, the biologically active molecule is a nucleic acid, preferably an miRNA.

In a preferred aspect, the biologically active molecule is selected from miR-21, an miR-21 mimic, an isomiR, an oligonucleotide molecule with more than 75% sequence similarity with miR-21, compounds intended for increasing the transcription or activity of miR-21 or any of the combinations thereof.

In a preferred aspect, the irradiation system that confers a negative charge comprises a heat supply source or an electric field generator, for example, laser or electroacupuncture.

In a preferred aspect, the biologically active molecule and the nanoparticle are attached to the surfactant by means of a covalent or non-covalent bond.

In a preferred aspect, the biologically active molecule and the nanoparticle are attached to the surfactant independently by means of a non-covalent bond which can be: electrostatic interactions, hydrophobic interactions, surface adsorption, encapsulation or intercalated inside same.

In a preferred aspect, the nanoparticle comprises a metal core.

In a preferred aspect, the metal core is a gold core.

In a preferred aspect, the mean size of the nanoparticles is comprised between 1 and 20 nanometers.

In a preferred aspect, the nanosystem is part of a pharmaceutical composition.

In a preferred aspect, the nanosystem is characterized in that its application comprises injecting the nanoparticle a) subcutaneously and performing irradiation b) on the same injection site.

In a preferred aspect, the biologically active molecule is selected from miR-21, an miR-21 mimic, an isomiR, an oligonucleotide molecule with more than 75% sequence similarity with miR-21, compounds intended for increasing the transcription or activity of miR-21 or any of the combinations thereof.

In a preferred aspect, the nanosystem is used for the treatment of obesity and/or type 2 diabetes.

For the interpretation of the present invention, the following terms are defined below:
- The term "comprising" means "including", but is not limited to what follows the expression "comprising". In that sense, the use of the term "comprising" indicates that the listed elements are necessary or mandatory, but that other elements are optional and may or may not be present.
- The term "consisting of" means "including", but is limited to what follows the expression "consists of". Therefore, the expression "consists of" indicates that the listed elements are mandatory and that no other elements may be present.
- "Biologically active substance" is understood to mean an active ingredient, an active substance or an active component having biological activity and being able to exert a therapeutic effect. miR-21 is used in the present invention as a proof of concept.

### Description of the Figures

**Figure 1****.** Body weight evolution in mice during 18 weeks of treatment using, as conduction methods, (A) the application of laser on the same puncture site (5 V for 25 seconds) (A) and (B) the application of electroacupuncture on the same puncture site (0.7 V for 25 seconds).
**Figure 2****.** miR-21 expression levels in the left inguinal fat (site of application of Au@16-ph-16/miR-21 mimic).
**Figure 3****.** miR-21 expression levels in the right inguinal fat of mice treated with Au@16-ph-16/miR-21 mimic and with the application of the two drive systems.
**Figure 4****.** miR-21 expression levels in the visceral fat of mice treated with Au@16-ph-16/miR-21 mimic and with the application of the two drive systems
**Figure 5****.** miR-21 expression levels in the interscapular brown fat of mice treated with the nanosystem and with the application of the two drive systems.
**Figure 6****.** miR-21 expression levels in the brain of mice treated with Au@16-ph-16/miR-21 mimic and with the application of the two drive systems.
**Figure 7****.** miR-21 expression levels in the heart of mice treated with Au@16-ph-16/miR-21 mimic and with the application of the two drive systems.
**Figure 8****.** miR-21 expression levels in the liver of mice treated with Au@16-ph-16/miR-21 mimic and with the application of the two drive systems.
**Figure 9****.** miR-21 expression levels in the spleen of mice treated with Au@16-ph-16/miR-21 mimic and with the application of the two drive systems.
**Figure 10****.** miR-21 expression levels in the kidney of mice treated with Au@16-ph-16/miR-21 mimic and with the application of the two drive systems.
**Figure 11****.** Thermogenesis and browning marker expression levels in the left inguinal fat of mice treated with Au@16-ph-16/miR-21 mimic and with the application of the two drive systems.
**Figure 12****.** Thermogenesis and browning marker expression levels in the right inguinal fat of mice treated with Au@16-ph-16/miR-21 mimic and with the application of the two drive systems.
**Figure 13****.** Thermogenesis and browning marker expression levels in the interscapular brown fat of mice treated with Au@16-ph-16/miR-21 mimic and with the application of the two drive systems.
**Figure 14****.** Mitochondrial DNA expression levels in the left inguinal fat of mice treated with Au@16-ph-16/miR-21 mimic and with the application of the two drive systems.
**Figure 15****.** Mitochondrial DNA expression levels in the right inguinal fat of mice treated with Au@16-ph-16/miR-21 mimic and with the application of the two drive systems

### Detailed Description of the Invention

The examples included below serve as a proof of concept to illustrate the invention without limiting the scope thereof.

### Example 1. Material and methods

### Example 1.1. Mice and diet-induced obesity

C57BL/6J mice (The Jackson Laboratory) were used to obtain obese phenotype after being fed a high-fat diet (HFD 45% kcal, D12451; Research Diets, New Brunswick, NJ, USA) for 8 weeks. The mice were housed individually in suitable temperature and humidity conditions and with free access to pelleted feed. Body weight was monitored two times a week. All the experimental protocols and methods were approved by the Ethics Committee of the University of Malaga (CEUMA Authorization No. 91-2021-A) and met European Union recommendations (2010/63/EU). The G*Power program was used to determine the suitable number of animals for this study.

### Example 1.2. Synthesis of Au@16-ph-16 precursor gold nanoparticles

Gold nanoparticle-functionalized Gemini surfactants were prepared by means of direct synthesis based on the *in situ* reduction of hydrogen tetrachloroaurate(III) hydrate (HAuCl₄·4H₂O) by means of the addition of NaBH₄ as a reducing agent and the suitable Gemini surfactant as a stabilizing agent. Determination of the ratio between AuNP/Gemini surfactant concentration and the critical micelle concentration (cmc) value of the surfactant is well known to be crucial for parameter stability and nanosystem size optimization. The [Gemini surfactant]/cmc ratio was equal to 5, with a lower limit and an upper limit being established at 1 and 20, respectively as a condition necessary for obtaining stable monodispersed nanosystems. 390 µl of a 23 mM aqueous HAuCl₄ solution were added to 30 ml of 4·10⁻⁵ M aqueous 16-Ph-16 Gemini surfactant solutions and it was stirred vigorously. The yellow solutions obtained were stirred for 5 minutes in the absence of light. Next, 100 µL of a freshly prepared 0.4 M aqueous NaBH₄ solution were added dropwise to the previously prepared solutions and the mixture was stirred moderately for 15 minutes in darkness, acquiring a reddish color. Aqueous Au@16-ph-16 gold nanoparticle solutions with concentrations of 5.6·10⁻⁸ M were obtained as a result.

### Example 1.3. Synthesis of Au@16-ph-16/miR-21 mimic gold nanoparticles

The Au@16-ph-16/miR-21 mimic was synthesized as described previously (J Mol Liquid Volume 313, 1 September 2020, 113577, https://doi.org/10.1016/j.molliq.2020.113577). Briefly, the synthesis of the nanosystems was stabilized with Gemini surfactants and functionalization with miR-21 was carried out using an excess of the polymer. The formulations were examined for the nanosystem, with fixed CAu@m-s-m = 5.6·10⁻⁹M for Au@16-ph-16 and variable CmiR-21 = 4.5·10⁻⁸ M, 6.0·10⁻⁸ M. As a result, different ratios R = CAu@m-s-m/CmiR-21 were examined in each system: R = 0.124, 0.093 and 0,063 for the Au@16-ph-16/miR-21 mimic. Complex Au@16-ph-16/miR-21 mimics which were stable after 30 minutes of continuously stirring the mixture at room temperature, and subsequently conditioning the obtained samples at a temperature of 277.0 K for 24 hours, were thus obtained. A moderate change in the color of the solution to a slightly purplish tone was indicative of complex stabilization.

### Example 1.4. In vivo treatment of mice with the Au@16-ph-16/miR-21 mimic nanosystem

After 8 weeks of feeding a HFD, obese mice were separated into 4 subgroups: (1) a group to which laser heating is applied (control), (2) a group to which an electric field is applied (control), (3) a group of mice which receive subcutaneous injections of 200 µl of 0.2 µg Au@16-ph-16/miR-21 mimic and to which laser is then applied, and (4) a group of mice which receive subcutaneous injections of 200 µl of 0.2 µg Au@16-ph-16/miR-21 mimic and to which electroacupuncture is then applied.

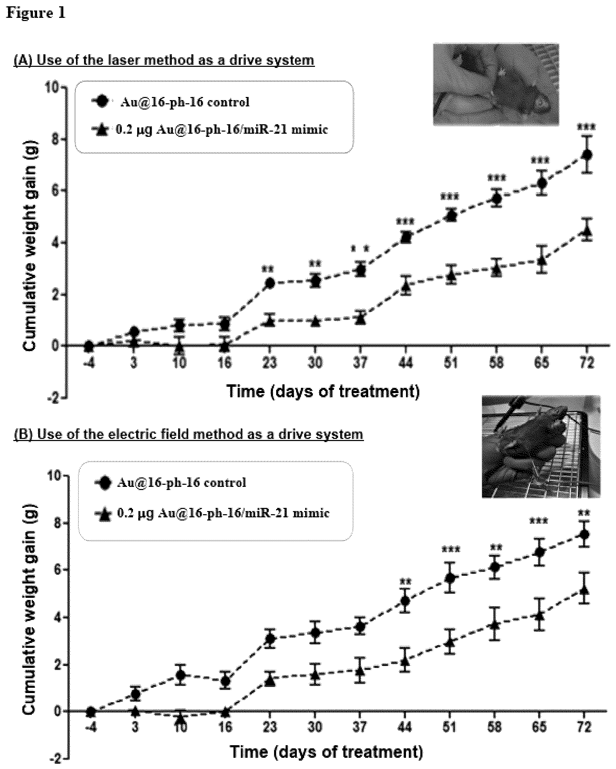

Body weight was monitored two times a week. Cumulative weight gain was calculated using the last pre-treatment weight for each mouse as a baseline point (0 g) (**Figure 1**).

### Example 1.5. Isolation of miRNAs from tissues and real-time quantitative PCR (qRT-PCR)

miRNA was extracted from tissues (80-100 mg) using the miRvana miRNA isolation kit (AM1561; Ambion, Spain) according to the manufacturer's recommendations. Total RNA was quantified using a Nanodrop ND-2000 (Thermo Fisher Scientific, USA). The reverse transcription reaction was carried out in a total volume of 15 µL, containing 5 µL (5 ng) of miRNA, 7 µL of the mixture consisting of RNase inhibitor, reverse transcriptase and buffer (Taqman microRNA reverse transcription, 4366597) and 3 µL of specific primer miRNAs, namely, hsa-miR-21 (000397) or control miRNA assay, snoRNA-142 (001231). qRT-PCR was performed using the Agilent Mx3005P qPCR system and previously indicated specific Taqman probes in a total volume of 20 µl. Cycle threshold (Ct) was determined during amplification and signals were normalized using snoRNA-142 as a reference gene.

### Example 1.6. Isolation of mRNA from adipose tissue and real-time quantitative PCR (qRT-PCR)

Total RNA was isolated from 100 mg of adipose tissue using the Qiazol RNeasy Lipid Tissue mini kit (QIAGEN, Valencia, CA, USA) according to the manufacturer's protocol. The RT reaction was performed using a reverse transcriptase (20 U/mL, 03531287001; Roche), RNase inhibitor (03335399001; Roche) and deoxynucleoside triphosphate (11969064001; Roche). qRT-PCR reactions were then performed using 10 ng of cDNA and specific probes. Next, the Ct of each gene was determined during amplification and the specific signals were then normalized using Tbp as a reference gene (Mm 00446973). Expression levels were calculated using the formula 2-^{Δct.}. The Taqman genes used in this study were: Ucp1 (Mm 01244861), Tmem26 (Mm 01173641-m1), Pgc-1a (Mm 01208835) and Prdm16 (Mm 00556-m1).

### Example 2. Results

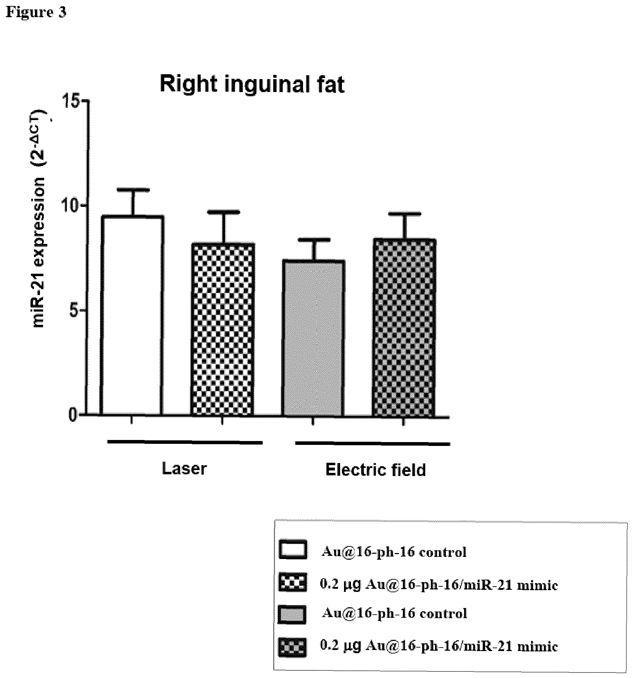

**Figure 2** highlights that the administration of the Au@16-ph-16/miR-21 mimic nanosystem, followed by the application of one of the drive systems, either laser or electric field, in the left inguinal region, resulted in a significant increase of miR-21 levels in said specific area. However, no changes in miR-21 levels were observed in the other tissues (such as right inguinal fat, visceral fat, interscapular brown fat, kidney, spleen, heart, and brain), suggesting that the nanosystem did not reach other regions (**Figures 3-10**).

These findings were supported by the analysis of the expression of thermogenesis and browning parameters in different fat deposits, including left inguinal fat (site of application of the treatment), right inguinal fat and interscapular fat. This analysis revealed a significant induction of the Prdm16, Tmem26, PGC1a and VEGA genes exclusively in left inguinal fat, while no changes whatsoever in the expression of these genes were observed in the other fat deposits (**Figures 11-15**).

## Claims

1. A nanosystem for use in capturing, directing and/or transporting biologically active molecules to adipose tissue, comprising:
(a) a positively-charged nanoparticle attached to a biologically active molecule, and
(b) an irradiation system that confers a negative charge specifically on adipose tissue, causing the nanoparticle to be specifically directed to and captured by the adipose tissue as a result of a charge difference.

2. The nanosystem for use according to claim 1, where the positively-charged nanoparticle is attached to the biologically active molecule through cationic surfactants that improve the uptake and stability of the biologically active molecule.

3. The nanosystem for use according to any of the preceding claims, where the biologically active molecule is a nucleic acid, preferably an miRNA.

4. The nanosystem for use according to any of the preceding claims, where the biologically active molecule is selected from miR-21, an miR-21 mimic, an isomiR, an oligonucleotide molecule with more than 75% sequence similarity with miR-21, compounds intended for increasing the transcription or activity of miR-21 or any of the combinations thereof.

5. The nanosystem for use according to any of the preceding claims, where the irradiation system that confers a negative charge comprises a heat supply source or an electric field generator, for example, laser or electroacupuncture.

6. The nanosystem for use according to any of the preceding claims, where the biologically active molecule and the nanoparticle are attached to the surfactant by means of a covalent or non-covalent bond.

7. The nanosystem for use according to any of the preceding claims, where the biologically active molecule and the nanoparticle are attached to the surfactant independently by means of a non-covalent bond which can be: electrostatic interactions, hydrophobic interactions, surface adsorption, encapsulation or intercalated inside same.

8. The nanosystem for use according to any of the preceding claims, where the nanoparticle comprises a cationic metal core.

9. The nanosystem for use according to any of the preceding claims, where the cationic metal core is a cationic gold core.

10. The nanosystem for use according to any of the preceding claims, where the mean size of the nanoparticles is comprised between 1 and 20 nanometers.

11. The nanosystem for use according to any of the preceding claims, **characterized in that** it is part of a pharmaceutical composition.

12. The nanosystem for use according to any of the preceding claims, **characterized in that** the method comprises injecting the nanoparticle a) subcutaneously and performing irradiation b) on the same injection site.

13. The nanosystem for use according to any of the preceding claims, in the treatment of obesity and/or type 2 diabetes.
